# EUROPEAN PATENT APPLICATION

(11) **EP 2 264 455 A1**
(43) Date of publication of application: **22.12.2010**
(21) Application number: 09163326.3
(22) Date of filing: 19.06.2009
(51) Int. Cl.: G01N 33/50

(54) **Method for the prediction of the severity of nuclear envelope-related diseases.**

(71) Applicant: Universiteit Maastricht, 6211 LK Maastricht (NL)
(72) Inventor: Broers, Joseph Leonardus Victor, 6267 BD Cadier en Keer (NL); Houben, Frederik, 3830 Wellen (BE); Ramaekers, Franciscus Charles Servatius, 6213 CB Maastricht (NL); Van Oostveldt, Patric Maurice V., 9000 Gent (BE); De Vos, Winnok Harald, 2610 Wilrijk (BE)
(74) Representative: Habets, Winand

(57) **Abstract**

Nuclear envelopathies are diseases caused by a malfunction of nuclear envelope associated proteins or lamins. Nuclear envelopathies are heterogeneous group of diseases, ranging from muscle and lipid dystrophies to progeria. Genetic screening does not warrant a proper assessment of the impact of the mutation(s) due to a highly variable penetrance between affected individuals. Here we present a novel method to assess and predict clinical outcome by screening the loss of nucleo-cytoplasmic compartmentalization in patient cells. Furthermore, this method allows distinguishing between different types of laminopathies.

SEQUENCE LISTING

<110>  Universiteit Maastricht

<120>  METHOD FOR THE PREDICTION OF THE SEVERITY OF NUCLEAR
       ENVELOPE-RELATED DISEASES

<130>  104 EPP0

<160>  3

<170>  PatentIn version 3.5

<210>  1
<211>  7
<212>  PRT
<213>  Artificial Sequence

<220>
<223>  NLS Signal Sequence

<400>  1

Pro Lys Lys Lys Arg Lys Val
1               5


<210>  2
<211>  16
<212>  PRT
<213>  Artificial Sequence

<220>
<223>  NLS signal sequence

<400>  2

Lys Arg Pro Ala Ala Thr Lys Lys Ala Gly Gln Ala Lys Lys Lys Lys
1               5                   10                  15


<210>  3
<211>  5
<212>  PRT
<213>  Artificial Sequence

<220>
<223>  NLS Signal Sequence

<400>  3

Lys Ile Pro Ile Lys
1               5

## Description

### Field of the invention

This invention is in the field of medical diagnostics and prognostics, specifically relating to a method for early detection of nuclear envelopathies and the prediction of the severity of this disease. Furthermore, this method allows distinguishing between different types of envelopathies, in particular laminopathies.

### Background of the invention

The nucleus of eukaryotic cells is separated from the cytoplasm by the nuclear envelope (NE). The NE is composed of three distinct elements, i.e. the nuclear membrane, nuclear pore complexes and the nuclear lamina (Gerace and Burke, 1988). The nuclear membrane is a double unit nuclear membrane, in which the outer nuclear membrane (ONM) its continuous with and shares biochemical and functional properties with the endoplasmic reticulum (ER). In contrast, the inner nuclear membrane (INM) is distinct from both the ONM and ER and is defined by a subset of integral membrane proteins, termed Nuclear Envelope Transmembrane proteins (NETs), that are anchored to the INM during interphase (Schirmer et al., 2003). The ONM and INM are separated by a luminal space of approximately 100nm in width. The nuclear membrane is punctuated by nuclear pore complexes (NPCs), which regulate the passage of macromolecules between the nucleus and the cytoplasm (Goldberg, 2004). At NPCs the ONM and INM converge at the so-called pore membrane, which again is defined by its own sub-set of integral membrane proteins (Hallberg et al., 1993; Wozniak et al., 1989). Underneath the INM lies the nuclear lamina. The principal components of the lamina are members of the lamin family of type V intermediate filament (IF) proteins (Fisher et al., 1986; McKeon et al., 1986).

It has recently been discovered that mutations in either proteins of the nuclear membrane or lamins or NPC proteins give rise to a wide range of inherited diseases, collectively termed nuclear envelopathies.

Mutations may arise in INM or NPC proteins (reviewed by (Burke et al., 2001; Nagano and Arahata, 2000; Somech et al., 2005) or in lamins (reviewed by Bonne et al., 2003; Burke and Stewart, 2002; Gruenbaum et al., 2005; Hutchison and Worman, 2004). In the latter case, these illnesses are collectively termed laminopathies.

Envelopathies include striated muscle disorders, partial lipodystrophy syndromes, peripheral neuropathies, progeroid syndromes and conditions that lead to severe developmental abnormalities or death *in utero.* Table 1 provides an overview of currently known envelopathies, more in particular laminopathies. For a more elaborate review see Broers et al., 2006; and Capell and Collins, 2006).

A striking feature of laminopathies, which account for the majority of NE related diseases, is that they are often caused by a single point mutation in the lamina A/C gene. However, this single mutation has an extremely variable impact on the pathophysiological development of the disease. Within family members who carry identical mutations, some people develop a severe laminopathy phenotype, resulting in early death due to heart failure, whereas siblings can remain disease-free. Thus, routine screening for genetic defects in the lamina A/C gene does not reliably predict instigation and/or development of the disease.

Clearly, the association of NE proteins with such a wide range of diseases implies important functions for these proteins in the normal development and/or maintenance of many different tissue types. Indeed many NE proteins have differential expression profiles during development and have been implicated in a range of cellular functions including the maintenance of cellular architecture, apoptosis, DNA replication and transcription (reviewed by (Hutchison, 2002)). Importantly, lamins which appear as a prominent rim-like labeling of the nuclear envelope using immunofluorescence, interact with and apparently stabilize several other structural NE proteins, such as emerin and nesprins. In addition, the appropriate distribution of nuclear pore complexes in the nuclear membrane is maintained by lamins (Hutchison, 2002).

In normal cells, a strict physical separation between nuclear and cytoplasm macromolecules is maintained by the nuclear envelope and the nuclear pore complex. Macromolecules with a physical size over 60kDa can normally only enter or leave the nucleus in interphase cells by active transport via the nuclear pore complex. Compartmentalization is critical for the functioning of cells: it ensures a correct and controlled localization of several transcription factors and signaling proteins

Nuclear envelopathies such as laminopathies are characterized by several morphological abnormalities of the nuclear envelope. Among these abnormalities are herniations, honeycomb-like structures and donut-shaped nuclei. Herniations are bleb-like expansions of the nuclear membrane, predicting weak spots in the nuclear lamina Honeycomb-like structures are characterized by abnormal distribution of chromatin and lamina-associated proteins leading to a reticulated nuclear appearance. Donut-shaped nuclei are nuclei with large intra-nuclear invaginations. While these abnormalities can be readily visualized, no clear correlation with severity of the disease has been established as yet.

**Table 1 Laminopathies that result from defects in the lamin A gene**

| **Disease** | **Mode of inheritance** | ***LMNA* defects** | **Effects on protein** | **Clinical phenotype** |
|---|---|---|---|---|
| Hutchinson-Gilford progeria syndrome | De novo mutations | 90% of cases due to C-to-T change at codon 608 in exon 11 of LMNA, activating a cryptic splice donor site | Creates a permanently farnesylated 'progerin' protein with 50 internally deleted amino acids near the C terminus | Premature ageing including alopecia, loss of subcutaneous fat and premature atherosclerosis; death in early teens |
| Atypical Werner syndrome | Autosomal dominant | Three reported mutations: A57P, R133L and L140R | Unknown; interactions with other proteins could be altered | Premature ageing beginning in the second decade; cataracts, sclerodermatous skin, premature atherosclerosis, hair greying |
| Emery-Dreifuss muscular dystrophy, type 2 | Autosomal dominant | Mutations reported in every codon except 12; most are missense | Misfolding or failure to assemble lamin A, leading to partial or complete loss of function | Slowly progressive contractures and muscle weakness; wasting of skeletal muscle and cardiomyopathy with conduction disturbances |
| Emery-Dreifuss muscular dystrophy, type 3 | Autosomal recessive | One reported case: H222Y | Unknown | Slowly progressive contractures and muscle weakness; wasting of skeletal muscle and cardiomyopathy with conduction disturbances |
| Limb girdle muscular dystrophy | Autosomal dominant | Six mutations described, three of which are missense | Unknown; one synonymous mutation leads to mutant lamin A with 15 additional amino acids | Slowly progressive shoulder and pelvic muscle weakness and wasting; later development of contractures and cardiac disturbances |
| Dilated cardiomyopathy, type 1A | Autosomal dominant | More than 20 mutations described, usually missense mutations in exons 1 or 3 | Unknown; head and rod domains usually affected | Ventricular dilatation, impaired systolic contractility, arrhythmias, conduction defects |
| Charcot-Marie-Tooth disease, type 2B1 | Autosomal recessive | Single R298C LMNA missense mutation in seven Algerian families | Rod domain affected; could affect lamin binding interactions | Lower-limb motor deficits, walking difficulty, secondary foot deformities and reduced or absent tendon reflexes in the second decade |
| Familial partial lipodystrophy, Dunnigan type | Autosomal dominant | Missense mutations cluster in exons 8 and 11, ∼75% of mutations in codon 482 of exon 8 | Globular domain affected; no effect on three-dimensional structure, but protein interactions could be altered | Loss of adipose tissue in the trunk and limbs with concomitant accumulation in the neck and face; often includes insulin-resistant diabetes, hypertriglyceridemia and increased susceptibility to atherosclerosis |
| Mandibuloacral dysplasia | Autosomal recessive usually; one compound heterozygote reported | R527H most common; K542N, A529V and heterozygous R527C/R471C (one case) also reported | Unknown; mutation affects the surface of the C-terminal domain that is common to both lamin A and C; could affect protein interactions | Delayed closure of cranial sutures, dental crowding, short stature, lipodystrophy, joint contractures, mandibular and clavicular hypoplasia, acroosteolysis, alopecia and insulin resistance |
| Restrictive dermopathy | De novo mutations in LMNA or recessive null mutations in ZMPSTE24 | Splicing mutations leading to partial or complete loss of exon11 (two patients); homozygous or compound heterozygous mutations in ZMPSTE24 could also be causative | LMNA mutation results in a prelamin A with a preterminal deletion; ZMPSTE24 mutations lead to abnormally aggregated lamin A and C and accumulation of prelamin A | Intrauterine growth retardation, tight and rigid skin erosions, microstomia, pulmonary hypoplasia; early lethality |
| Generalized lipoatrophy/ lipodystrophy | Autosomal dominant | Single cases of R133L and T101 mutations | Unknown | Variable phenotypic overlap with other laminopathies with generalized lipodystrophy or lipoatrophy; other features include diabetes, hypertriglyceridemia and progeroid features |

| | | | | |
|---|---|---|---|---|
| Data obtained from Capell and Collins, 2006 | | | | |

It has also been described to assess nuclear membrane abnormalities by performing mechanical loading on cells. Laminopathy cells showed a significant loss of mechanical strength in experimental setups (Broers et al., 2004; Lammerding et al., 2004). However, these experiments are difficult to perform and are not suitable in routine screening setups.

### Summary of the invention

We herein present a number of markers for correct cellular compartmentalization that are indicative of nuclear membrane integrity and/or disease severity and which can be determined in a routine laboratory setting.

The present invention therewith relates to a method for determining the state of nucleo-cytoplasmic compartmentalization wherein the presence of nuclear markers outside of the nucleus is determined.

In more detail, this in vitro method may be used in the diagnosis of nuclear envelopathies, for predicting the severity of a nuclear envelopathy, or for selecting a drug against a Nuclear envelopathy.

Hence the invention relates to a method for diagnosing a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a disease related to an envelopathy if the percentage obtained in step b is at least 5% higher than the normal percentage.

More in particular, the invention relates to a method for predicting the severity of a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a severe envelopathy if the percentage obtained in step b is more than 15 % higher than the normal percentage and that the patient is suffering from a mild disease if the percentage obtained in step b is between 5 % to 15% higher than the normal percentage.

Even more in particular, the invention relates to a method for selecting a drug effective in curing a nuclear envelopathy comprising the steps of:
a. Providing cells obtained from an individual suspected to suffer from a nuclear envelopathy and exposing the cells to a candidate drug
b. Determining the percentage of living cells containing a nuclear marker in the cytoplasm or a cytoplasmic marker in the nucleus before and after the exposure of the cells to the candidate drug,
c. Comparing the percentage thus obtained with a normal percentage
wherein it is concluded that the drug may be effective in curing a nuclear envelopathy when the percentage obtained after exposure is lower as compared to the percentage obtained before exposure.

### Detailed description of the invention

Using a selection of dermal fibroblast cell cultures from patients with different types of envelopathies, we have observed that a highly variable loss of nucleo-cytoplasmic compartmentalization occurs, depending on the type of disease and its severity.

We have determined the occurrence of a number of nuclear markers in cell compartments outside the nucleus, such as the cytoplasm and found that patients with nuclear envelopathies may be distinguished from normal individuals by determining the percentage of cells wherein nuclear markers appear in the cytoplasm or wherein cytoplasmic markers appear in the nucleus.

Hence the invention relates to a method for diagnosing a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a disease related to an envelopathy if the percentage obtained in step b is at least 5% higher than the normal percentage.

A normal percentage is to be understood as the percentage of living cells containing a nuclear marker in the cytoplasm or a cytoplasmic marker in the nucleus when the method is performed on cells obtained from a normal individual.

In healthy control cells, nuclear markers were found in the cytoplasm in about 0% of the cells in most cases. Cytoplasmic markers in the nucleus were also found in about 0% of the cells in most cases. In those cases where nuclear markers were found in the cytoplasm of normal cells or wherein cytoplasmic markers were found in the nucleus of normal cell, this percentage was never above 3% of the cells.

In patients with mild disease, Nuclear markers were observed in the cytoplasm of 8 - 18% of the cells. The same percentages were found for cytoplasmic markers in the nucleus. Patients with severe disease showed nuclear markers in the cytoplasm or cytoplasmic markers in the nucleus in more than 18% of the cells.

Hence, the invention relates to a method for predicting the severity of a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a severe envelopathy if the percentage obtained in step b is more than 15 % higher than the normal percentage and that the patient is suffering from a mild disease if the percentage obtained in step b is between 5 % to 15% higher than the normal percentage.

The invention may also be exploited as a method for screening candidate drugs for their efficacy against nuclear envelopathies. When a drug is capable of decreasing the leakage of nuclear markers from the nucleus or decreasing the leakage of cytoplasmic markers into the nucleus, it is considered a promising lead candidate for drug development.

Therefore, the invention relates to a method for selecting a drug effective in curing a nuclear envelopathy comprising the steps of:
a. Providing cells obtained from an individual suspected to suffer from a nuclear envelopathy and exposing the cells to a candidate drug
b. Determining the percentage of living cells containing a nuclear marker in the cytoplasm or a cytoplasmic marker in the nucleus before and after the exposure of the cells to the candidate drug,
c. Comparing the percentage thus obtained with a normal percentage
wherein it is concluded that the drug may be effective in curing a nuclear envelopathy when the percentage obtained after exposure is lower as compared to the percentage obtained before exposure.

The above methods may advantageously be employed in nuclear envelopathies such as Hutchinson-Gilford progeria syndrome, Atypical Werner syndrome, Emery-Dreifuss muscular dystrophy type 2, Emery-Dreifuss muscular dystrophy type 3, Limb girdle muscular dystrophy , Dilated cardiomyopathy type 1A, Charcot-Marie-Tooth disease type 2B1, Familial partial lipodystrophy Dunnigan type, Mandibuloacral dysplasia, Restrictive dermopathy and Generalized lipoatrophy/ lipodystrophy,

The above methods may also advantageously be used when the nuclear envelopathy is a nuclear laminopathy.

The cells that are used in the above methods may be any cells obtained from a human or animal individual. The methods may be employed on primary cells directly obtained from the individual; they may also be cultured before they are used in the methods according to the invention. The cells may be obtained as individual cells or as tissue sections. In some cases it may be advantageous to obtain cells from tumour tissue, particularly preferred however are dermal fibroblasts.

The nuclear or cytoplasmic markers that are used in the methods according to the invention may be any marker, such as a protein or nucleic acid that is specifically localized in the nucleus or cytoplasm. Particularly useful nuclear markers were found to be PML bodies ,NLS-containing proteins, nucleic acid markers, Cajal bodies, nuclear stress granules and SC35 also known as nuclear speckles. Particularly useful cytoplasmic markers were found to be cyclins, eIF3, also known as cytoplasmic stress granule and heat shock factor proteins.

The nuclear or cytoplasmic marker may also be a marker that is artificially introduced into the cell. An artificial marker in this context is a marker that does not normally appear in the cell but has been introduced by an external action such as microinjection, transfection, electroporation, electropermeabilization, cell fusion, introduction of foreign DNA and/or protein and any other method known in the art for introducing markers into a living cell. A particularly useful artificial marker appeared to be a protein comprising a green fluorescence protein attached to a nuclear localization signal. The artificial marker used in the experimental setion is a green fluorescent protein fused to a polypeptide comprising sequence (SEQ ID NO: 1). The marker was introduced as a DNA vector into the living cell by electroporation.

So-called PML bodies appeared to be a very suitable nuclear marker for this purpose. PML bodies are well known in the art. They are also known as nuclear dots, nuclear bodies, or nuclear domains. PML bodies are punctate structures found in the nucleus of certain cells. PML bodies were first seen as prominent interchromatin structures in the nuclei of malignant or hyperstimulated animal cells. PML bodies may be identified using anti-sp100 autoantibodies from primary biliary cirrhosis. PML bodies appeared to be elevated in many autoimmune and cancerous diseases. PML bodies are metabolically stable and resistant to nuclease digestion and salt extraction.

Simple PML bodies (types I and II) and the shells of complex PML bodies (types III, IVa and V) consist of a non-chromatin fibrillar material, which is most likely proteinaceous. PML bodies could be co-isolated with the nuclear matrix, and were linked to the fibrogranular nuclear matrix component by projections from the surface of the PML bodies The primary components of PML bodies are the proteins sp100 nuclear antigen, LYSP100 (a homolog of sp100), , ISG20, PML antigen, NDP55 and 53kDa protein associated with the nuclear matrix. Other proteins, such as PIC1/SUMO-1, which are associated with nuclear pore complex also associate with PML bodies. The proteins can reorganize in the nucleus, by increasing number of dispersion in response to different stress such as stimulation or heat shock.

One of the PML body proteins appears to be involved in transcriptional active regions. Expression of PML antigen and sp100 is responsive to interferons. Sp100 seems to have transcriptional transactivating properties. PML protein was reported to suppress growth and transformation and specifically inhibits the infection of vesicular stomatitis virus (VSV) (a rhabdovirus) and influenza A virus, but not other types of viruses.

The SUMO-1 ubiquitin like protein is responsible for modifying PML protein such that it is targeted to nuclear bodies whereas overexpression of PML results in programmed cell death. One hypothesized function of the dots is as a 'nuclear dump' or 'storage depot'. The nuclear bodies may not all perform the same function. Sp140 associates with certain bodies and appears to be involved in transcriptional activation.

PML bodies or similar bodies have been found increased in the presence of lymphoid cancers and Systemic Lupus Erythematosus. They are also observed at higher frequencies in subacute sclerosing panencephalitis in these instances antibodies to measles shows expression and localization to the bodies.

In Promyelocytic Leukemia the oncogenic PML-RARalpha chimera disrupts normal concentration of PML into nuclear bodies. Addition of As203, retenoic acid causes remission of this leukemia by triggering their reorganization. As203 destroys the chimera, allowing new SUM0-1 ubiquitinated PML to relocalize to nuclear bodies. Retinoic acid induces a capsase-3 mediated degradation of the same chimera.

Cells lacking lamin A/C expression showed a loss of nucleo-cytoplasmic compartmentalization in up to 70% of all cells, based on PML nuclear body immunostaining. This high level of compromised nuclear envelope structures corresponds well with high levels of morphologically detectable nuclear abnormalities. In contrast, classic (HGPS) progeria cells did not show any loss of PML nuclear bodies from the nucleus. This can be attributed to the fact that progeria is characterized by stiffening of the nuclear membrane due to accumulation of unprocessed mutant lamin proteins. Indeed, these cells appear to be mechanically stronger than their lamin A/C negative counterparts (Verstraeten et al., 2008).

Comparison of these data indicate that presence of extranuclear PML nuclear bodies can be used to detect laminopathies and distinguish between different types of laminopahies

In a parallel study we assessed the nucleo-cytoplasmic compartmentalization in living cells in time using another nuclear marker GFP-tagged Nuclear Localisation Signal (NLS), a chimeric protein destined to become actively transported into the nucleus after synthesis.

A nuclear localization signal or sequence (NLS) is an amino acid sequence which acts like a 'tag' on the exposed surface of a protein. This sequence is used to target the protein to the cell nucleus through the Nuclear Pore Complex and to direct a newly synthesized protein into the nucleus via its recognition by cytosolic nuclear transport receptors. Typically, this signal consists of one or more short sequences of positively charged lysines or arginines. Different nuclear localized proteins may share the same NLS. An NLS has the opposite function of a nuclear export signal, which targets proteins out of the nucleus.

The first NLS discovered is the sequence PKKKRKV (SEQ ID NO: 1) in SV40 large T antigen. The NLS of nucleoplasmin, KR[PAATKKAGQA]KKKK (SEQ ID NO: 2) is the prototype of ubiquitous bipartite signal: two clusters of basic amino acids, separated by a spacer of about 10 amino acids. Both signals are recognized by importin α. Importin α contains a bipartite NLS itself, which is specifically recognized by importin β. The latter can be considered the actual import mediator.

A proposed consensus sequence for monopartite NLS is suggested by Dingwall C et al., 1988. This is often referred to as a Chelsky sequence. This consensus sequence may therefore be part of the downstream basic cluster of a bipartite NLS. Makkerh et al. carried out comparative mutagenesis on the nuclear localisation signals of SV40 T-Antigen (monopartite), C-myc (monopartite) and nucleoplasmin (bipartite), and showed amino acid features common to all three. Notably the role of neutral and acidic amino acids was shown for the first time in contributing to the efficiency of the NLS (Makkerh JP et al., '1996).

There are many other types of NLS, such as the acidic M9 domain of hnRNP A1, the sequence KIPIK (SEQ ID NO: 3) in yeast transcription repressor Matα2, and the complex signals of U snRNPs. Most of these NLSs appear to be recognized directly by specific receptors of the importin β family without the intervention of an importin α-like protein.

A signal that appears to be specific for the massively produced and transported ribosomal proteins seems to come with a specialized set of importin β-like nuclear import receptors.

A protein translated with a NLS will bind strongly to importin (aka karyopherin), and together, the complex will move through the nuclear pore. At this point, Ran-GTP will bind to the importin-protein complex, and its binding will cause the importin to lose affinity for the protein. The protein is released, and now the Ran-GTP/importin complex will move back out of the nucleus through the nuclear pore. A GTPase activating protein (GAP) in the cytoplasm hydrolyzes the Ran-GTP to GDP, and this causes a conformational change in Ran, ultimately reducing its affinity for importin. Importin is released and Ran-GDP is recycled back to the nucleus where guanine exchange factor (GEF) exchanges its GDP back for GTP.

While in normal interphase cells the GFP-tagged NLS protein is exclusively localized in the nucleus, it will diffuse out of the nucleus upon rupture of the nuclear envelope. In cell cultures of lamin A/C deficient cells we observed such a rupture in a considerable part of the cells, while in such a rupture never occured in normal dermal fibroblasts. From these studies we conclude that the loss of nucleo-cytoplasmic compartmentalization is a common event in laminopathy cells.

We found that the integrity of the nuclear envelope, in particular of the lamina correlates well with the severity of the disease. The rupture of the NE when measured in the ways as described above predicts a worse phenotype in affected patients with respect to especially gene regulation, cell differentiation and proliferation.

In summary, nuclear envelopathies are diseases caused by a malfunction of nuclear envelope associated proteins or lamins. Nuclear envelopathies are heterogeneous group of diseases, ranging from muscle and lipid dystrophies to progeria. Genetic screening does not warrant a proper assessment of the impact of the mutation(s) due to a highly variable penetrance between affected individuals. Here we present a novel method to assess and predict clinical outcome by screening the loss of nucleo-cytoplasmic compartmentalization in patient cells. We have observed that patients with cells wherein a severe loss of nucleo-cytoplasmic compartmentalization could be determined, have a less favourable prognosis as compared to patients with less affected or unaffected cells.

### Legend to the figures

### Figure 1

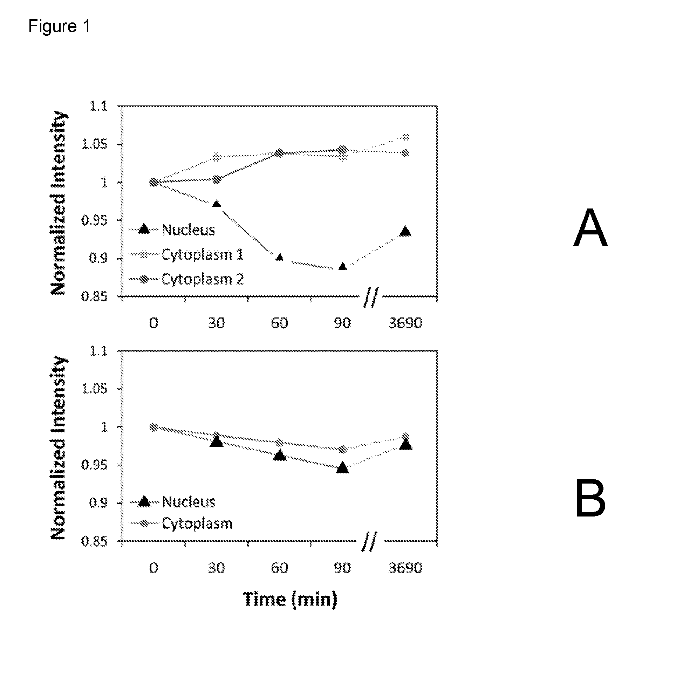

Figure 1 shows the normalized intensity of the fluorescence in the nucleus and at two different locations at five different time intervals. It can be seen that the normalized fluorescence in the nucleus of A-type lamin deficient cells obtained from an individual with a nuclear envelopathy (panel A) reaches a minimum at 90 minutes and then gradually returns to its normal value after 3690 minutes, whereas the normalized fluorescence of the cytoplasm increases slightly over time but essentially remains constant. Panel B shows the control experiment performed on fibroblast cells obtained from a normal individual. No temporary loss of signal is observed here.

### Examples

### Example 1: Immunstaining for PML and counterstaining with DAPI in cell cultures

Patient fibroblasts were obtained from dermal biopsy material. Cells were cultured and grown on glass coverslips. The cells were then fixed for 15 minutes with 3,7% paraformaldehyde in PBS and permeabilized with 0,1% Triton-X-100. Next, the cells were stained with the primary mouse anti-PML antibody (sc-966, Santa Cruz, Heidelberg, Germany) in a 1:1000 dilution and the primary rabbit anti-lamin C antibody (RaIC, (MUbio Products BV, Maastricht, the Netherlands) in a 1:200 dilution. As secundary antibodies, GAR-TxRed and GAM-Ig-FITC were used in a 1:50 dilution. Cells were mounted in Tris-buffered glycerol containing DABCO and DAPI. The number of cells demonstrating PML body staining in the cytoplasm was scored using a fluorescence microscope, by determining the localization of PML nuclear bodies within or outside of the nuclear lamina, which was visualized by the lamina C antibody staining. Quantitation was either performed by manual scoring of 4x 100 cells, or using high throughput screening (automated image acquisition and analysis for statistically relevant percentages.

It was found that PML bodies were excusively localized in the nucleus in normal fibroblast cells. They were visible as bright fluorescent spots outside of the nucleus, indicating that PML bodies can be seen in the cytoplasm of living cells lacking lamin A/C. Cells were counterstained with a lamin B antibody and with DAPi in order to visualize the contours of the nucleus.

### Example 2: Immunstaining for PML in tissue sections.

Frozen or paraffin-embedded material obtained from dermal biopsy material was used for the detection of PML nucear bodies in patient tissues. 4 to 7 µm frozen sections were air dried and fixed in cold (-20°C) methanol. After incubation with the primary antibody, followed by several washes with PBS, slides were incubated with rabbit anti-mouse IgG conjugated to peroxidase (DAKOpatts, Glostrup, Denmark), at a dilution of 1:50 in PBS containing 5% human AB serum. After a second series of washes, peroxidase activity was detected using 3-amino-9-ethylcarbazole (0.2 mg/ml in 0.05 mol/L sodium acetate buffer, pH 4.8). Sections were briefly counterstained with hematoxylin to determine the exact localization of PML bodies in relation to the nucleus, and mounted with Kaiser's medium (Merck, Darmstadt, Germany

### Example 3: Dynamics of GFP-NLS in living cells

When an artificial marker comprising a green fluorescent protein attached to a nuclear localization signal (SEQ ID NO: 1) was introduced into a living cell, we observed a temporary loss of signal in A-type lamin deficient cells. This is quantified in figure 1

### References

1. Bonne, G. et al., (2003). Neuromuscul Disord 13, 508-15.
2. Broers, J.L. et al., (2004). Hum Mol Genet 13, 2567-80.
3. Broers, J.L. et al., (2006). Physiol Rev 86, 967-1008.
4. Burke B., et al., (2001). Traffic 2, 675-83.
5. Burke, B. and Stewart, C. L. (2002). Nat Rev Mol Cell Biol 3, 575-85.
6. Capell, B. C and Collins, F. S. (2006) Nat Rev Genet 7, 940-52.
7. Fisher, D. Z et al., (1986). Proc. Natl. Acad. Sci. USA 83, 6450-6454.
8. Gerace, L. and Burke, B. (1988). Annu Rev Cell Biol 4, 335-74.
9. Goldberg, M. (2004). Symp Soc Exp Biol, 115-33.
10. Gruenbaum, Y., et al., (2005). Nat Rev Mol Cell Biol 6, 21-31.
11. Hallberg, E., et al., (1993).J Cell Biol 122, 513-21.
12. Hutchison, C. J. (2002). Nat Rev Mol Cell Biol 3, 848-58.
13. Hutchison, C. J. and Worman, H. J. (2004). Nat Cell Biol 6, 1062-7.
14. Lammerding, J., et al., (2004). J Clin Invest 113, 370-8.
15. McKeon, F. D., et al., (1986).Nature (London) 319, 463-468.
16. Nagano, A. and Arahata, K. (2000). Curr Opin Neurol 13, 533-9.
17. Schirmer, E. C., et al., (2003). Science 301, 1380-2.
18. Somech, R., et al., (2005). Pediatr Res 57, 8R-15R.
19. Verstraeten, V. L., et al., (2008). Aging cell 7, 383-393.
20. Wozniak, R. W., et al.,(1989). J Cell Biol 108, 2083-92.
21. Dingwall C et al., 1988, J Cell Biol 107 (3): 841-9.
22. Makkerh JP et al., 1996, Curr Biol. 6 (8): 1025-7.

## Claims

1. A method for diagnosing a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a disease related to an envelopathy if the percentage obtained in step b is at least 5% higher than the normal percentage.

2. A method for predicting the severity of a nuclear envelopathy comprising the steps of:
a. Providing cells obtained from an individual suspected to suffer from a nuclear envelopathy,
b. Determining the percentage of living cells containing a nuclear marker in the cytoplasm or a cytoplasmic marker in the nucleus,
c. Comparing the percentage thus obtained with a normal percentage
wherein it is concluded that the patient suffers from a severe envelopathy if the percentage obtained in step b is more than 15 % higher than the normal percentage and that the patient is suffering from a mild disease if the percentage obtained in step b is between 5 % to 15% higher than the normal percentage.

3. A method for selecting a drug effective in curing a nuclear envelopathy comprising the steps of:
a. Providing cells obtained from an individual suspected to suffer from a nuclear envelopathy and exposing the cells to a candidate drug
b. Determining the percentage of living cells containing a nuclear marker in the cytoplasm or a cytoplasmic marker in the nucleus before and after the exposure of the cells to the candidate drug,
c. Comparing the percentage thus obtained with a normal percentage
wherein it is concluded that the drug may be effective in curing a nuclear envelopathy when the percentage obtained after exposure is lower as compared to the percentage obtained before exposure.

4. A method according to claims 1 - 3 wherein the nuclear envelopathy is selected from the group consisting of Hutchinson-Gilford progeria syndrome, Atypical Werner syndrome, Emery-Dreifuss muscular dystrophy type 2, Emery-Dreifuss muscular dystrophy type 3, Limb girdle muscular dystrophy , Dilated cardiomyopathy type 1A, Charcot-Marie-Tooth disease type 2B1, Familial partial lipodystrophy Dunnigan type, Mandibuloacral dysplasia, Restrictive dermopathy and Generalized lipoatrophy/ lipodystrophy,

5. A method according to claims 1 - 3 herein the nuclear envelopathy is a nuclear laminopathy.

6. A method according to claims 1 - 5 wherein the cells are cultured *in vitro* after being obtained from an individual.

7. A method according to claims 1 - 6 wherein the cells are dermal fibroblasts.

8. A method according to claims 1 - 7 wherein the cells are obtained in tissue sections.

9. A method according to claims 1 - 8 wherein the cells are obtained from tumor tissue.

10. A method according to claims 1 - 9 wherein the nuclear marker is selected from the group consisting of PML bodies ,NLS-containing proteins, nucleic acid markers, Cajal bodies nuclear stress granules and SC35 also known as nuclear speckles.

11. A method according to claims 1 - 9 wherein the cytoplasmic marker is selected from the group consisting of cyclins, eIF3, also known as cytoplasmic stress granule and heat shock factor proteins

12. A method according to claims 1 - 11 wherein the nuclear marker is an artificial protein such as a protein comprising a green fluorescence protein attached to a nuclear localization signal.
